# EUROPEAN PATENT APPLICATION

(11) **EP 4 793 266 A1**
(43) Date of publication of application: **19.08.2026**
(21) Application number: 24942061.3
(22) Date of filing: 30.10.2024
(51) Int. Cl.: C07C 57/26

(54) **ARTEMISINIC ACID DERIVATIVE, AND PREPARATION METHOD THEREFOR AND USE THEREOF**

(30) Priority: 20.09.2024 CN 202411316507
(71) Applicant: Yunnan Botanee Bio-Technology Group Co., Ltd., Kuming, Yunnan 650106 (CN); Yunnan Yunke Characteristic Plant Extraction Laboratory Co., Ltd., Kunming, Yunnan 650106 (CN)
(72) Inventor: WANG, Yichun, Kunming, Yunnan 650106 (CN); WANG, Feifei, Kunming, Yunnan 650106 (CN); QU, Liping, Kunming, Yunnan 650106 (CN); SHEN, Yanzhen, Kunming, Yunnan 650106 (CN); WANG, Zuding, Kunming, Yunnan 650106 (CN); WU, Qiongfen, Kunming, Yunnan 650106 (CN); YAN, Lijuan, Kunming, Yunnan 650106 (CN); YANG, Zhaoxiang, Kunming, Yunnan 650106 (CN); MA, Xiao, Kunming, Yunnan 650106 (CN); GUO, Zhenyu, Kunming, Yunnan 650106 (CN)
(74) Representative: Cabinet Chaillot
(86) International application number: PCT/CN2024/128309
(87) International publication number: WO 2026/060778

(57) **Abstract**

An artemisinic acid derivative includes: a water-soluble organic salt formed by a reaction of artemisinic acid or dihydroartemisinic acid with a small-molecule basic organic compound, or a water-soluble complex formed by a reaction of artemisinic acid or dihydroartemisinic acid with a small molecule peptide, or a water-soluble salt formed by a reaction of artemisinic acid or dihydroartemisinic acid with a basic metal compound. The artemisinic acid derivative provided by the present application solves the problem of difficulty in dissolving the artemisinic acid and the dihydroartemisinic acid in water, and combines whitening, anti-inflammatory, and anti-tumor biological activities, thus having a good research and development prospect.

## Description

### CROSS-REFERENCE TO RELARED APPLICATIONS

This application is a continuation of International Application No. PCT/CN2024/128309, filed on October 30, 2024, which claims priority to Chinese Patent Application No. 202411316507.8, filed on September 20, 2024. All of the aforementioned applications are incorporated herein by reference in their entireties.

### FIELD OF TECHNOLOGY

The present application relates to the technical field of biomedicine, and particularly relates to an artemisinic acid derivative, and a preparation method therefor and an application thereof.

### BACKGROUND

Human skin pigmentation is associated with multiple factors, such as pigment metabolism, inflammation, and skin barrier impairment, thus being a highly complex process. A pigment metabolism disorder is also influenced by both internal factors of the human body and external environments, but all these influences lead to a common symptom: inflammation, and the inflammation also exacerbates the pigment metabolism disorder. Therefore, single regulation is difficult to address the pigmentation issue. Different degrees of pigment deposition show different clinical symptoms, such as freckles, chloasma, senile plaques, and pigment deposition caused by inflammation. Melanin can prevent the skin, hair, and eyes of humans from being damaged by ultraviolet light, but excessive melanin leads to the pigment deposition on the skin surface to form color spots, and even diseases such as melanoma. Tyrosinase is a key rate-limiting enzyme in generation of melanin, and inhibiting its activity can improve skin diseases related to excessive melanin. Substances with an inhibition effect on the tyrosinase include mercury compounds, hydroquinone, ascorbic acid, and glutathione, etc.

Among the substances, the mercury compounds, although having obvious whitening effects, are highly toxic and prone to deposition in the body, which may cause internal organ failure of the human body. The hydroquinone has severe skin irritation. Thiol compounds, such as the glutathione and cysteine, not only have pungent odors, but also have an issue of transdermal absorption. The ascorbic acid is easily oxidized in a state of an aqueous solution and cannot exert an effect continuously.

Artemisia annua contains abundant artemisinic acid with a content of 8-10 times of a content of artemisinin, and has pharmacological activities in many aspects, such as bacterial resistance, heat relief, and tumor resistance. However, the Artemisia annua is discarded during extraction of the artemisinin, resulting in severe resource waste. Studies have shown that artemisinic acid can reduce the melanin by inhibiting expression of an HMGCoA reductase gene. However, artemisinic acid has poor water solubility, and its activity intensity also needs to be further improved.

Therefore, providing an active substance with excellent spot-removing and whitening effects and higher safety has a good application prospect.

### SUMMARY

To solve the above technical problems, the present application provides an artemisinic acid derivative, and a preparation method thereof and an application thereof. The artemisinic acid derivative provided by the present application solves the problem of difficulty in dissolving artemisinic acid in water, and combines excellent whitening and anti-tumor biological activities, thus having a good research and development prospects.

To achieve the purpose, the present application adopts the following technical solutions.

In a first aspect, the present application provides an artemisinic acid derivative, where the artemisinic acid derivative includes: a water-soluble organic salt formed by a reaction of artemisinic acid or dihydroartemisinic acid with a small-molecule basic organic compound, or a water-soluble complex formed by a reaction of artemisinic acid or dihydroartemisinic acid with a small molecule peptide, or a water-soluble salt formed by a reaction of artemisinic acid or dihydroartemisinic acid with a basic metal compound; and the artemisinic acid derivative has the following structures: 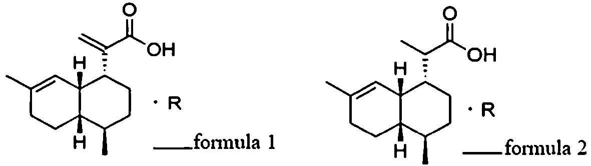 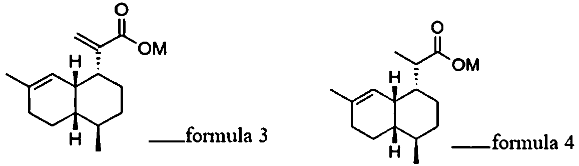 where R is selected from a small-molecule basic organic compound or a small molecule peptide, and M is a metal.

The artemisinic acid derivative provided by the present application not only overcomes the defect of poor water solubility of the artemisinic acid, but also further enhances biological activities, has an excellent tyrosinase inhibition effect, can inhibit growth of mouse melanoma cells, combines whitening and anti-tumor activities, is non-toxic to normal cells, and has high safety, thus having a good research and development prospect.

Preferably, the small-molecule basic organic compound includes a basic amino acid or an alkaloid.

Preferably, the basic amino acid includes lysine, arginine, or histidine.

Preferably, the alkaloid includes nicotinamide or ligustrazine.

Preferably, the small molecule peptide includes glutathione.

Preferably, the basic metal compound includes a sodium salt, a potassium salt, sodium hydroxide, or potassium hydroxide.

Preferably, the sodium salt includes any one or a combination of two of sodium carbonate or sodium bicarbonate.

Preferably, the potassium salt includes any one or a combination of two of potassium carbonate or potassium bicarbonate.

Preferably, the artemisinic acid derivative is selected from any one of the following compounds:

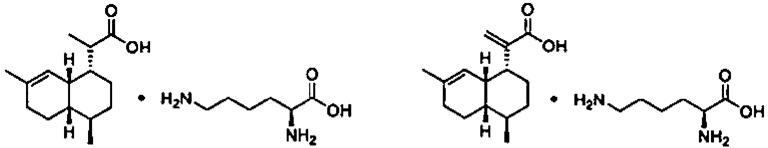

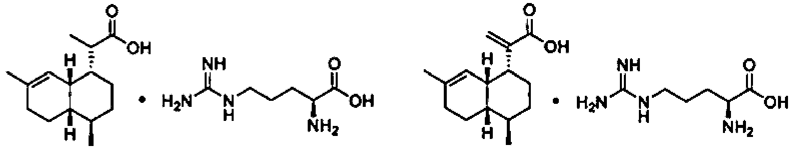

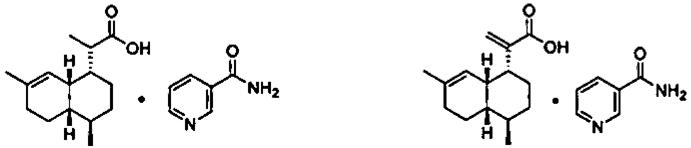

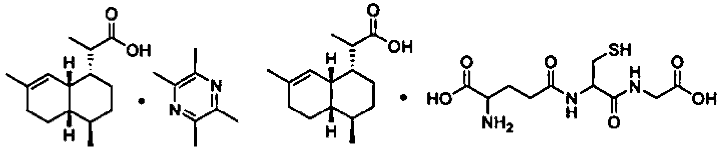

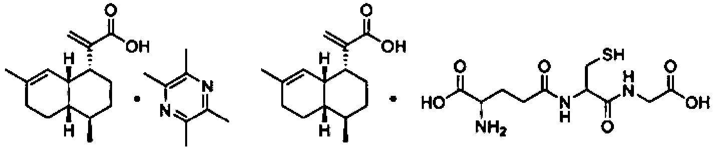

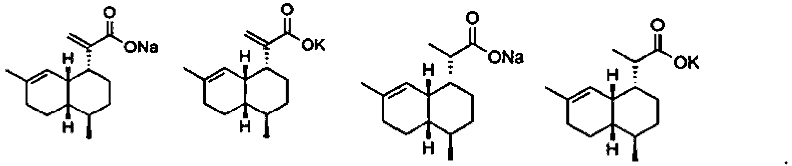

In a second aspect, the present application provides a method for preparing the artemisinic acid derivative according to the first aspect, which includes: allowing the artemisinic acid or the dihydroartemisinic acid to undergo a reaction with a modifier in water to obtain the artemisinic acid derivative, where the modifier is a small-molecule basic organic compound, a small molecule peptide, or a basic metal compound.

Preferably, a molar ratio of the artemisinic acid or the dihydroartemisinic acid to the modifier is 1:(0.9-1.1), and may be, for example, 1:0.92, 1:0.95, 1:0.98, 1:1, 1:1.02, 1:1.05, and 1:1.08, etc.

Preferably, a use amount of the water is 5-20 times of a total mass of the artemisinic acid or the dihydroartemisinic acid and the modifier, and may be, for example, 6, 8, 10, 12, 15, and 18, etc.

Preferably, a temperature of the reaction is 20-30°C, and may be, for example, 22°C, 24°C, 25°C, 26°C, and 28°C, etc.

Preferably, a time of the reaction is 30-60 s, and may be, for example, 35 s, 40 s, 45 s, 50 s, and 55 s, etc.

Preferably, the method further includes a step of post-treatment after the reaction is completed, and the post-treatment includes filtration and drying.

Preferably, the drying includes any one or a combination of at least two of freeze-drying, spray drying, or vacuum drying.

In a third aspect, the present application provides an application of the artemisinic acid derivative according to the first aspect in a skin care product or a pharmaceutical product.

When the artemisinic acid derivative provided by the present application is used for preparing the skin care product or the pharmaceutical product, the skin care product or the pharmaceutical product can be endowed with excellent spot-removing and whitening effects and an anti-inflammatory effect.

Preferably, the skin care product includes an essence liquid, a facial mask, a microneedle, an emulsion, a lyophilized powder, or a face cream.

Preferably, a mass percentage of the artemisinic acid derivative in the skin care product is 1-40%, and may be, for example, 2%, 5%, 10%, 15%, 20%, 25%, 30%, and 35%, etc.

Preferably, a dosage form of the pharmaceutical product includes a tablet, a capsule, a granule, an injection, a spray, or a film.

In a fourth aspect, the present application provides an emulsion, where the emulsion includes one or a combination of at least two of the artemisinic acid derivative according to the first aspect, an emollient, an emulsifier, a thickener, a stabilizer, a preservative, and an antioxidant.

In a fifth aspect, the present application provides a lyophilized powder, where the lyophilized powder includes one or a combination of at least two of the artemisinic acid derivative according to the first aspect, and a humectant.

In a sixth aspect, the present application provides a tyrosinase inhibitor, where the tyrosinase inhibitor includes any one or a combination of at least two of the artemisinic acid derivative according to the first aspect.

Compared with the prior art, the present application at least has the following beneficial effects.

The artemisinic acid derivative provided by the present application not only overcomes the defect of poor water solubility of the artemisinic acid, but also further enhances biological activities, has an excellent tyrosinase inhibition effect, can inhibit growth of mouse melanoma cells, combines whitening, anti-inflammatory, and anti-tumor activities, is non-toxic to normal cells, and has high safety, thus having a good research and development prospect.

### BRIEF DESCRIPTION OF THE DRAWINGS

FIG. 1 is a diagram of high performance liquid chromatography (HPLC) results of artemisinic acid.
FIG. 2 is a diagram of HPLC results of dihydroartemisinic acid.
FIG. 3 is a diagram of thin-layer chromatography analysis in Example 1.
FIG. 4 is a diagram of thin-layer chromatography analysis in Example 2.
FIG. 5 is a diagram of thin-layer chromatography analysis in Example 3.
FIG. 6 is a diagram of thin-layer chromatography analysis in Example 4.
FIG. 7 is a diagram of thin-layer chromatography analysis in Example 5.
FIG. 8 is a diagram of thin-layer chromatography analysis in Example 6.
FIG. 9 is a diagram of thin-layer chromatography analysis in Example 7.
FIG. 10 is a schematic diagram of cytotoxicity test results of the artemisinic acid and its derivatives.
FIG. 11 is a schematic diagram of whitening activity evaluation results of the artemisinic acid and its derivatives.
FIG. 12 is a cell culture image of the artemisinic acid and its derivatives.
FIG. 13 is a diagram of content determination of anti-inflammatory factor NO.
FIG. 14 is a diagram of content determination of anti-inflammatory factor IL-6.
FIG. 15 is a diagram of content determination of anti-inflammatory factor TNF-α.

### DETAILED DESCRIPTION OF THE EMBODIMENTS

The technical solutions of the present application are further illustrated below through specific embodiments in conjunction with the accompanying drawings. However, the following examples are merely simple instances of the present application and do not represent or limit the scope of protection of the present application, and the scope of protection of the present application is defined by the claims.

Reagents, materials, and instruments used in the following examples can all be obtained through commercial means.

### Experimental Example 1

### Mass study of artemisinic acid

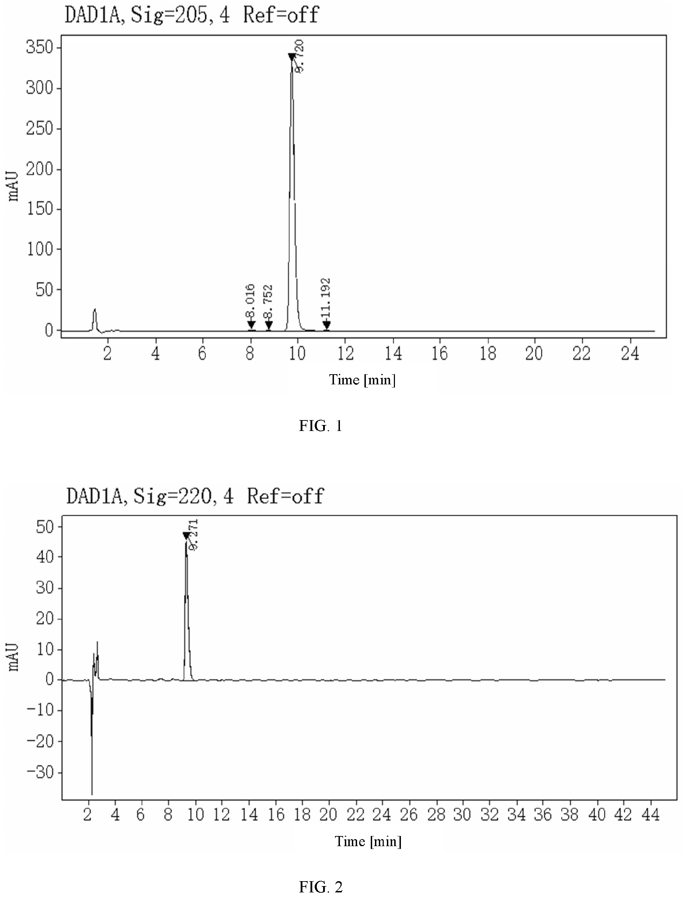

Artemisinic acid (HPLC ≥ 98%) was dissolved in methanol using a C18 (4.6 nm × 250 nm, 5 µm) chromatographic column at a temperature of 35°C and a flow rate of 1.0 mL/min under ultraviolet (UV) at 203 nm. Chromatographic conditions were adopted as follows: phase A: acetonitrile; phase B: 0.1% phosphoric acid aqueous solution; and elution procedure: at 0-20 min, phase A: 37%, and phase B: 63%; and at 20-25 min, phase A: 37-90%, and phase B: 63-10%. Results were processed, and a purity of the artemisinic acid was calculated based on a peak area. Analysis results are shown in Table 1 and FIG. 1.

**Table 1**

| Retention time/min | Peak area | Peak height | Peak area proportion/% |
|---|---|---|---|
| 8.016 | 15.96 | 1.35 | 0.34 |
| 8.752 | 8.75 | 0.61 | 0.19 |
| 9.72 | 4629.3 | 333.77 | 99.3 |
| 11.192 | 7.71 | 0.5 | 0.17 |

As shown in the above table, according to the peak area, it can be known that the purity of artemisinic acid is 99.3%, which is greater than 98%.

### Experimental Example 2

### Mass study of dihydroartemisinic acid

Dihydroartemisinic acid (HPLC ≥ 98%) was dissolved in methanol using a C18 (4.6 nm × 150 nm, 5 µm) chromatographic column at a temperature of 35°C and a flow rate of 1.0 mL/min under UV at 203 nm. Chromatographic conditions were adopted as follows: phase A: acetonitrile; phase B: 0.1% phosphoric acid aqueous solution; and elution procedure: at 0-15 min, phase A: 56%, and phase B: 44%; and at 15-18 min, phase A: 56-95%, and phase B: 44-5%. Results were processed, and a purity of the dihydroartemisinic acid was calculated based on a peak area. Analysis results are shown in Table 2 and FIG. 2.

**Table 2**

| Retention time/min | Peak area | Peak height | Peak area proportion/% |
|---|---|---|---|
| 9.271 | 690.1396 | 45.631 | 98.07 |

As shown in the above table, according to the peak area, it can be seen that the purity of dihydroartemisinic acid is 98.07%, which is greater than 98%.

### Example 1

### Nicotinamide artemisinate (derivative 2) and a preparation method

Nicotinamide artemisinate has a structural formula as follows:

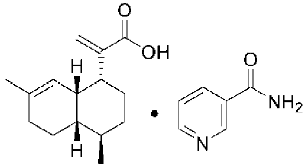

A preparation method is as follows: adding 2.44 g of nicotinamide into 100 mL of distilled water for stirring until being dissolved, adding 4.68 g of artemisinic acid in portions under stirring conditions to carry out a complete reaction under stirring (to form a clear solution), and performing filtration to obtain the clear solution after the reaction of the artemisinic acid and the nicotinamide; and removing the solvent by evaporation using a rotary evaporator, then performing vacuum drying at 60°C to obtain a solid, and grinding the solid into a powder to obtain the nicotinamide artemisinate (4.3 g, yield: 93%).

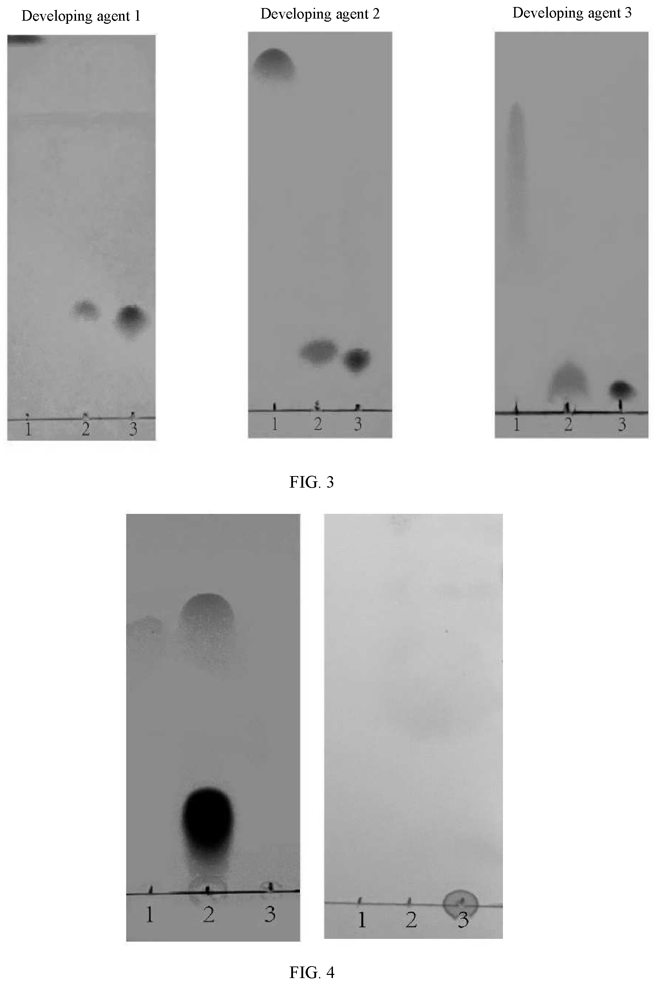

Thin-layer chromatography analysis results of the derivative 2 are shown in FIG. 3, in which 1 represents the artemisinic acid, 2 represents the derivative 2, and 3 represents the nicotinamide; and developing agent 1 includes petroleum ether and ethyl acetate at a ratio of 2:3, developing agent 2 includes petroleum ether and acetone at a ratio of 5:3, and developing agent 3 includes petroleum ether and dichloromethane at a ratio of 2:1, where all the above ratios refer to volume ratios.

The results indicate that different developing agent systems all show that the artemisinic acid and the nicotinamide react to generate the derivative 2.

### Example 2

### Glutathione artemisinate (derivative 3) and a preparation method

Glutathione artemisinate has a structural formula as follows:

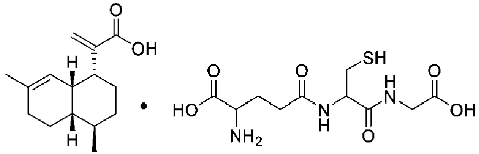

A preparation method is as follows: adding 6.14 g of glutathione into 100 mL of distilled water for stirring until being dissolved, adding 4.68 g of artemisinic acid in portions under stirring conditions to carry out a complete reaction under stirring (to form a clear solution), and performing filtration to obtain the clear solution after the reaction of the artemisinic acid and the glutathione; and using a spray dryer with an inlet temperature set at 125°C and an air outlet temperature of 60°C to obtain a white powder, that is, the glutathione artemisinate (4.2 g, yield: 89%).

Thin-layer chromatography analysis of the derivative 3 is shown in FIG. 4, in which 1 represents the artemisinic acid, 2 represents the derivative 3, 3 represents the glutathione, and a developing agent system includes petroleum ether, ethyl acetate, methanol, and water at a volume ratio of 1:4:1:0.2.

### Example 3

### Lysine artemisinate (derivative 5) and a preparation method

Lysine artemisinate has a structural formula as follows:

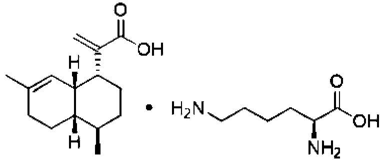

A preparation method is as follows: adding 2.92 g of lysine into 100 mL of distilled water for stirring until being dissolved, adding 4.68 g of artemisinic acid in portions under stirring conditions to carry out a complete reaction under stirring (to form a clear solution), and performing filtration to obtain the clear solution after the reaction of the artemisinic acid and the lysine; and using a spray dryer with an inlet temperature set at 135°C and an air outlet temperature of 70°C to obtain a white powder, that is, the lysine artemisinate (3.9 g, yield: 83%).

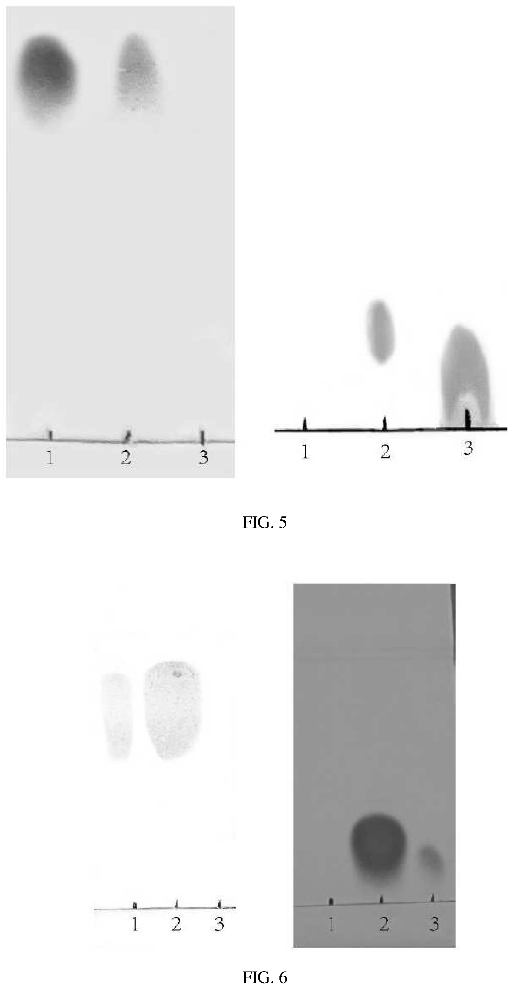

Thin-layer chromatography analysis of the derivative 5 is shown in FIG. 5, in which 1 represents the artemisinic acid, 2 represents the derivative 5, 3 represents the lysine, and a developing agent system includes ethyl acetate, methanol, and water at a volume ratio of 1:4:1.

### Example 4

### Nicotinamide dihydroartemisinate (derivative 6) and a preparation method

Nicotinamide dihydroartemisinate has a structural formula as follows:

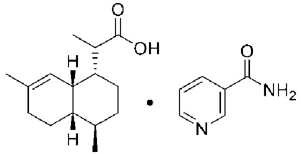

A preparation method is as follows: adding 2.44 g of nicotinamide into 100 mL of distilled water for stirring until being dissolved, adding 4.73 g of dihydroartemisinic acid in portions under stirring conditions to carry out a complete reaction under stirring (to form a clear solution), and performing filtration to obtain the clear solution after the reaction of the dihydroartemisinic acid and the nicotinamide; and removing the solvent by evaporation using a rotary evaporator, then performing spray drying to obtain a solid, and grinding the solid into a powder to obtain the nicotinamide dihydroartemisinate (4.1 g, yield: 86%).

Thin-layer chromatography analysis results of the derivative 6 are shown in FIG. 6, in which 1 represents the dihydroartemisinic acid, 2 represents the derivative 6, 3 represents the nicotinamide, and a developing agent system includes petroleum ether and ethyl acetate at a volume ratio of 2:3.

### Example 5

### Glutathione dihydroartemisinate (derivative 7) and a preparation method

Glutathione dihydroartemisinate has a structural formula as follows:

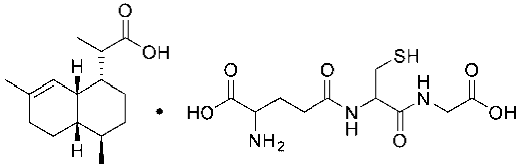

A preparation method is as follows: adding 6.14 g of glutathione into 100 mL of distilled water for stirring until being dissolved, adding 4.73 g of dihydroartemisinic acid in portions under stirring conditions to carry out a complete reaction under stirring (to form a clear solution), and performing filtration to obtain the clear solution after the reaction of the dihydroartemisinic acid and the glutathione; and removing the solvent by evaporation using a rotary evaporator, then performing vacuum pressure drying to obtain a solid, and grinding the solid into a powder to obtain the glutathione dihydroartemisinate (4.5 g, yield: 95%).

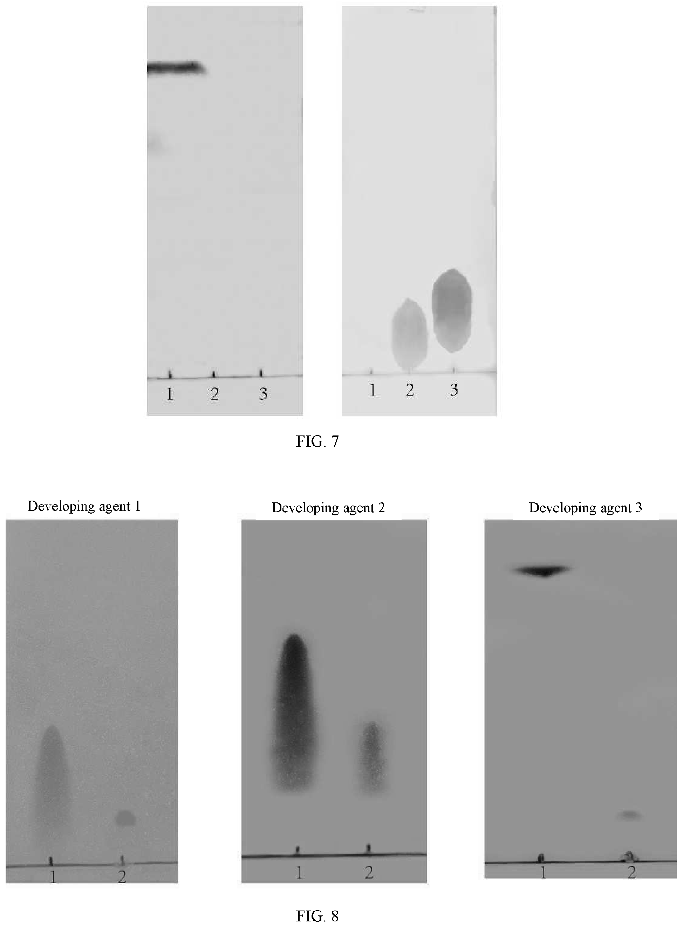

Thin-layer chromatography analysis of the derivative 7 is shown in FIG. 7, in which 1 represents the dihydroartemisinic acid, 2 represents the derivative 7, 3 represents the glutathione, and a developing agent system includes ethyl acetate, methanol, and water at a volume ratio of 1:4:2.

### Example 6

### Sodium artemisinate (derivative 1) and a preparation method

Sodium artemisinate has a structural formula as follows:

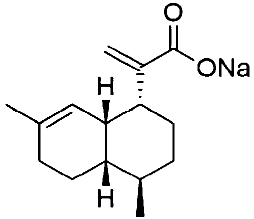

1.6 g of sodium bicarbonate was added into 100 mL of distilled water and stirred until being dissolved, 4.68 g of artemisinic acid was added in portions under stirring conditions to carry out a complete reaction under stirring (to form a clear solution), and filtration was performed to obtain a sodium artemisinate solution. Drying was performed using a vacuum low-temperature drying method, cryogenic freezing was performed at -40°C, vacuum drying was performed, and then the solution was gradually heated to 55°C for a total time of 16 h to obtain white porous and loose sodium artemisinate (4.5 g, yield: 96%).

Thin-layer chromatography analysis results of the derivative 1 are shown in FIG. 8, in which 1 represents the artemisinic acid, and 2 represents the derivative 1; and developing agent 1 includes petroleum ether and dichloromethane at a ratio of 2:3, developing agent 2 includes petroleum ether and ethyl acetate at a ratio of 1:1, and developing agent 3 includes n-hexane and ethyl acetate at a ratio of 2:3, where all the above ratios refer to volume ratios.

From the results, it can be seen that in different developing agent systems, the two react to generate the derivative 1. Since the sodium bicarbonate is an inorganic compound, it does not show peaks in thin-layer chromatography.

### Example 7

### Potassium dihydroartemisinate (derivative 4) and a preparation method

Potassium dihydroartemisinate has a structural formula as follows:

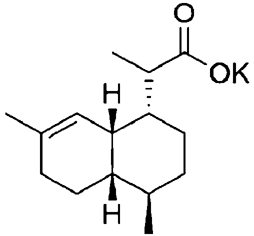

2.00 g of potassium bicarbonate was added into 100 mL of distilled water and stirred until being dissolved, 4.73 g of dihydroartemisinic acid was added in portions under stirring conditions to carry out a complete reaction under stirring (to form a clear solution), and filtration was performed to obtain a potassium dihydroartemisinate solution. The solvent was removed by evaporation using a rotary evaporator, and then vacuum drying was performed at 55°C to obtain a white powder of potassium dihydroartemisinate (4.6 g, yield: 97%).

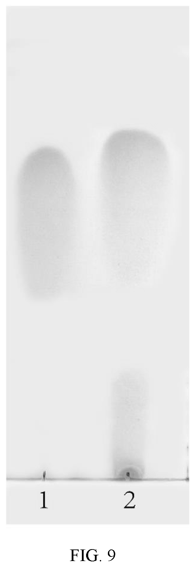

Thin-layer chromatography analysis results of the derivative 4 are shown in FIG. 9, in which 1 represents the dihydroartemisinic acid, 2 represents the potassium dihydroartemisinate, and a developing agent includes petroleum ether and ethyl acetate at a volume ratio of 2:3.

### Application Example 1

### A liquid preparation (emulsion) and a preparation method

This application example provides an emulsion, where raw materials for preparation of the emulsion include: 25 g of an artemisinic acid derivative, 10 g of an emollient, 5 g of an emulsifier, 1.5 g of a thickener, 0.05 g of a stabilizer, 0.15 g of a preservative, 0.01 g of an antioxidant, and 78.28 g of water. The emollient is glycerol, the emulsifier is glyceryl stearate, the thickener is xanthan gum, the stabilizer is EDTA-2Na, the preservative is phenoxyethanol, and the antioxidant is sodium pyrosulfite.

A method for preparing the emulsion includes: evenly mixing the water, the emollient, the stabilizer, and the preservative, heating up to 80°C, then adding the emulsifier under stirring for homogenization for 8 min, subsequently, adding the thickener under stirring, then lowering the temperature to 35°C, and subsequently, adding the artemisinic acid derivative 2 and the antioxidant for uniform stirring to obtain the emulsion.

### Application Example 2

### A solid preparation (lyophilized powder) and a preparation method

This application example provides a lyophilized powder, where raw materials for preparation of the lyophilized powder include: 31 g of an artemisinic acid derivative, 20 g of mannitol, 5 g of trehalose, and 65 g of deionized water.

A method for preparing the lyophilized powder includes: dissolving and evenly mixing the above raw materials, filtering the raw materials through 0.45 µm and 0.22 µm filter elements, respectively, adding the raw materials into a container, plugging the container, and then placing the container in a vacuum freeze dryer for pre-freezing at -40°C for 2.5 h, vacuumizing to 0.19 mbar, and heating for sublimation drying for 26 h until water is completely sublimated, followed by pressure plugging and sealing under vacuum conditions. During use, a powder dissolving solution is sterile water or normal saline.

### Application Example 3

### A whitening essence and a preparation method

This application example provides a whitening essence, where contents of various ingredients are respectively as follows: 0.25% of EDTA-2Na, 0.6% of p-hydroxyacetophenone, 5% of dipropylene glycol, 0.4% of 1,2-hexanediol, 0.1% of sodium hyaluronate, 0.05% of panthenol, 25% of a derivative, and the balance of water.

A method for preparing the whitening essence includes: mixing the water, the dipropylene glycol, the EDTA-2Na, the 1,2-hexanediol, and the sodium hyaluronate, heating up to 85°C, and performing heat preservation and stirring until being completely dissolved evenly to obtain an aqueous-phase liquid; lowering the temperature to 65°C, adjusting a rotation speed to 220 r/min, and adding the p-hydroxyacetophenone; and when the temperature is lowered to 45°C, adding the panthenol and the derivative 2 for uniform mixing to obtain a whitening essence liquid.

### Test Example 1

### Sensitization safety assessment - ADRA experiment

Test samples: artemisinic acid and derivatives 1-7.

### Test method:

On the day of the experiment, a positive control and a test compound solution were formulated at a concentration of 1 mmol/L, where the positive control was phenylacetaldehyde with a purity of above 90%. Solvent selection: Acetonitrile, water, acetonitrile/water (V:V=1:1), isopropanol, acetone, acetone/acetonitrile (V:V=1:1), and other solvents not affecting peptide stability were selected. If a compound was still insoluble, the compound was tried to be sequentially dissolved in 300 µL of dimethyl sulfoxide (DMSO) and diluted with 2,700 µL of acetonitrile, or dissolved in 1,500 µL of DMSO and diluted with 1,500 µL of acetonitrile. A test compound and a derivative were mixed to carry out a reaction in the dark for 24±1 h at a temperature of 25±1°C, determination by HPLC was performed within 1 h after the reaction was completed, and all tests were completed within 30 h. Before and after the reaction, a sample injection vial was observed, and whether a precipitate occurred and other situations were recorded. When the precipitate occurred before the reaction started, a consumption percentage of the derivative cannot be calculated, a positive result is available, and a negative result is uncertain. When the precipitate occurred only after the reaction, centrifugation was performed at a low rotation speed of 100-400 g to collect the precipitate at a bottom of the sample injection vial, followed by sample injection. $\begin{matrix}Consumption percentage of derivative \\ = \left(1-\frac{Mean peak area of sample derivative}{Mean peak area of solvent control derivative}\right) \times 100 %\end{matrix}$

### Judgment criteria:

(1) When the test compound does not undergo co-elution with both a cysteine derivative and a lysine derivative, a 1:50 cysteine derivative and 1:50 lysine derivative determination model as shown in Table 3 is used for determination.

**Table 3**

| Mean consumption percentage of NAC and NAL | Prediction result |
|---|---|
| 0% ≤ mean consumption percentage < 4.9% | Negative |
| 4.9% ≤ mean consumption percentage ≤ 100% | Negative |

(2) When the test compound undergoes co-elution only with the lysine derivative, a 1:50 cysteine derivative model as shown in Table 4 is used for determination.

**Table 4**

| Mean consumption percentage of NAC | ADRA prediction result |
|---|---|
| 0% ≤ mean consumption percentage < 5.6% | Negative |
| 5.6% ≤ mean consumption percentage ≤ 100% | Negative |

Sensitization results of the artemisinic acid are shown in Table 5.

**Table 5**

| Sample name | Consumption of NAC | Consumption of NAL | Mean consumption value | Prediction result |
|---|---|---|---|---|
| Artemisinic acid | 0.36% | 0% | 0.18% | Negative |
| Dihydroartemisinic acid | 0% | 0% | 0% | Negative |
| Derivative 2 | 0% | 1.18% | 0.59% | Negative |
| Derivative 3 | 1.08% | 0 | 0.54% | Negative |
| Derivative 5 | 7.71% | 0.71% | 4.21% | Negative |
| Derivative 6 | 0% | 0% | 0% | Negative |
| Derivative 7 | 2.11% | 1.41% | 1.76% | Negative |
| Derivative 1 | 4.41% | 0% | 2.2% | Negative |
| Derivative 4 | 0% | 0% | 0% | Negative |

The test results show that the artemisinic acid, the dihydroartemisinic acid, and their derivatives all have negative sensitization results, indicating higher safety.

### Test Example 2

### Determination of cytotoxicity of artemisinic acid and its derivatives by an MTT assay

Test samples: artemisinic acid and derivatives 1-7.

### Test method:

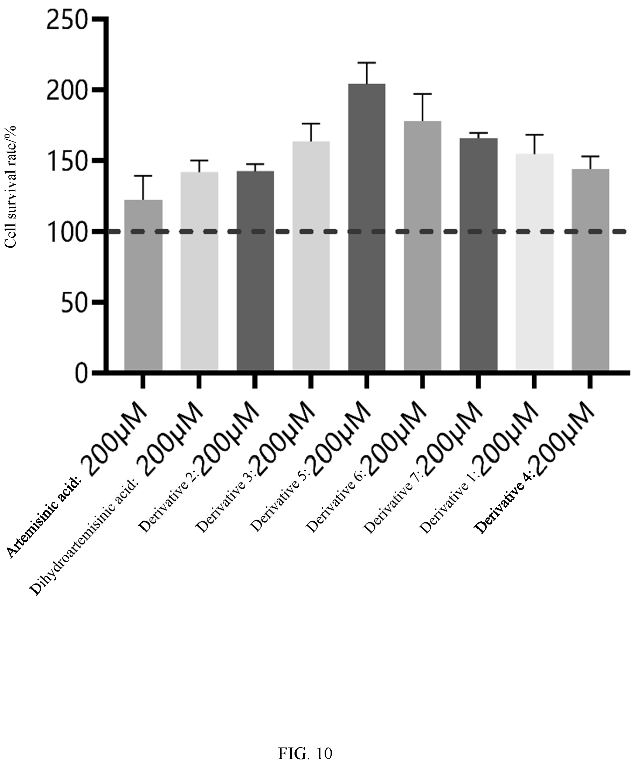

B16F10 cells in a good growth state were selected, digested, and added into a DMEM culture medium containing 10% of FBS to prepare a cell suspension, and the cells were inoculated into a 96-well plate at a concentration of 1×10⁵ cells/mL and placed in a 5% CO₂ incubator for culture at 37°C for 24 h. Then, the artemisinic acid and its derivatives (at a concentration of 200 µM) were added. Meanwhile, a control group and a blank group were set, with 3 replicate wells set for each group. The cells were continuously cultured at 37°C under 5% CO₂ for 24 h, then 25 µL of MTT was added for continued culture for 2-4 h, the culture medium was rinsed off, DMSO was added at 150 µL/well, shaking was performed for 10 min, and an absorbance value was measured at a wavelength of at 570 nm using a microplate reader. Results are shown in FIG. 10 and Table 6.

**Table 6**

| Sample | Cell survival rate/% |
|---|---|
| Artemisinic acid (200 µM) | 122.3 |
| Dihydroartemisinic acid (200 µM) | 142 |
| Derivative 2 (200 µM) | 142.7 |
| Derivative 3 (200 µM) | 163.7 |
| Derivative 5 (200 µM) | 204.7 |
| Derivative 6 (200 µM) | 178 |
| Derivative 7 (200 µM) | 166 |
| Derivative 1 (200 µM) | 154.7 |
| Derivative 4 (200 µM) | 144.3 |

From the MTT assay results in FIG. 10 and Table 6, it can be known that after 6 concentrations of drugs are adopted for intervention, growth and development of the cells are promoted, indicating that both the artemisinic acid and its derivatives in the present application have no toxic effect on the B16F10 cells, thus proving that the obtained products have the characteristic of low toxicity and have greatly improved safety.

### Test Example 3

### Determination of a tyrosinase inhibition effect

Test samples: artemisinic acid and derivatives 1-7.

### Test method 1:

With L-tyrosine as a catalytic substrate for monophenolase, 200 µL of tyrosinase as well as 20 µL of inhibitors of different concentrations (0 mM, 0.625 mM, 1.25 mM, 2.5 mM, 5 mM, and 10 mM) and PBS (added into a blank group) were sequentially added into a 96-well plate. After heat preservation at 37°C for 10 min, 50 µL of the L-tyrosine was added to carry out a reaction for 5 min, and the plate was placed in a microplate reader to measure an absorbance value at 475 nm. Plotting was performed with an inhibitor concentration as the abscissa and an inhibition rate as the ordinate to calculate an IC50 value. A positive control was α-arbutin. Results are shown in Table 7.

**Table 7**

| Sample | Inhibition rate/% |
|---|---|
| α-arbutin (10 mM) | 65 |
| Derivative 2 (10 mM) | 82 |
| Derivative 3 (10 mM) | 93 |
| Derivative 5 (10 mM) | 72 |
| Derivative 6 (10 mM) | 64 |
| Derivative 7 (10 mM) | 89 |
| Artemisinic acid (10 mM) | 56 |
| Derivative 1 (10 mM) | 65 |
| Derivative 4 (10 mM) | 51 |

From Table 7, it can be seen that the derivatives 3 and 7 have a great tyrosinase inhibition effect better than the α-arbutin, and after the derivatives 3 and 7 are formed, water solubility of the artemisinic acid and the dihydroartemisinic acid is further enhanced, indicating that a combination of the glutathione with the artemisinic acid or the dihydroartemisinic acid is synergistic. In addition, an inhibition rate of the derivative 2 is also higher than that of the artemisinic acid acting separately.

### Test method 2:

With L-DOPA as a catalytic substrate for diphenolase, 50 µL of L-DOPA as well as 20 µL of inhibitors of different concentrations (0, 0.625 mM, 1.25 mM, 2.5 mM, 5 mM, and 10 mM) and PBS (added into a blank group) were sequentially added into a 96-well plate. Then, 200 µL of tyrosinase was added for a reaction at room temperature for 30 min, and the plate was placed in a microplate reader to measure an absorbance value at 475 nm. Plotting was performed with an inhibitor concentration as the abscissa and an inhibition rate as the ordinate to calculate an IC50 value. A positive control was α-arbutin. Results are shown in Table 8.

**Table 8**

| Sample | Inhibition rate/% |
|---|---|
| α-arbutin (10 mM) | 75 |
| Derivative 2 (10 mM) | 84 |
| Derivative 3 (10 mM) | 97 |
| Derivative 5 (10 mM) | 66 |
| Derivative 6 (10 mM) | 68 |
| Derivative 7 (10 mM) | 90 |
| Artemisinic acid (10 mM) | 58 |
| Derivative 1 (10 mM) | 61 |
| Derivative 4 (10 mM) | 63 |

From Table 8, it can be seen that the derivatives 3 and 7 have a great tyrosinase inhibition effect better than the α-arbutin, and after the derivatives 3 and 7 are formed, water solubility of the artemisinic acid and the dihydroartemisinic acid is further enhanced, indicating that a combination of the glutathione with the artemisinic acid or the dihydroartemisinic acid is synergistic. The inhibition effect is not changed after the action substrate is changed, indicating that the derivatives have a stable inhibition effect. It is indirectly indicated that the artemisinic acid, the dihydroartemisinic acid, and their derivatives can play a whitening role by inhibiting the tyrosinase.

### Test Example 4

### Evaluation of whitening activity of artemisinic acid and its derivatives (cell experiment)

Test samples: artemisinic acid and derivatives 1-7.

### Test method:

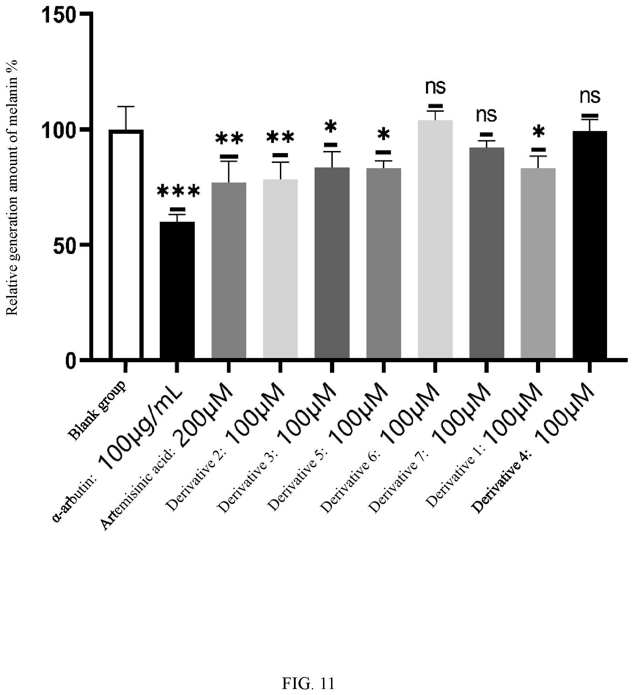

B16F10 cells in a state of an exponential growth phase were selected, digested with trypsin, and inoculated into a 6-well plate at a cell density of 7×10⁴ cells/mL at 2 mL/well. After adherent culture overnight, a culture medium was replaced, and the cells were treated with the artemisinic acid and its derivatives for 48 h, with 3 replicate wells for each sample. After the culture medium was discarded, the cultured cells were rinsed once with PBS. Cell culture dishes were placed on an ice plate, 330 µL of a non-denaturing cell lysis buffer (containing 1mM of PMSF) was added into each dish for lysis at 4°C for 20 min, and the cells were collected. Centrifugation was performed at 13,000 r/min for 10 min to collect melanin precipitates at bottoms of centrifuge tubes. 330 µL of NaOH (containing 10% of DMSO) was added for vortexing to facilitate complete lysis, and the cells were placed in a metal bath for lysis at 80°C for 2 h to fully dissolve the melanin precipitates. Vortexing was performed for uniform mixing, 200 µL of a melanin dissolving solution was added into each well of a 96-well plate, 3 replicate wells and a blank well were set, and an OD value was measured at 405 nm. Test results are shown in FIG. 11 and Table 9. $\begin{matrix}Relative content of melanin \\ = \frac{OD value of sample group - OD value of blank group}{OD value of control group - OD value of blank group} \times 100 %\end{matrix}$

**Table 9**

| Sample | Relative generation amount of melanin/% |
|---|---|
| Blank group | 100 |
| α-arbutin (100 µg/mL) | 60.14 |
| Derivative 2 (200 µM) | 78.31 |
| Derivative 3 (200 µM) | 81.57 |
| Derivative 5 (200 µM) | 83.27 |
| Derivative 6 (200 µM) | 103.92 |
| Derivative 7 (200 µM) | 92.16 |
| Artemisinic acid (200 µM) | 77.10 |
| Derivative 1 (200 µM) | 83.26 |
| Derivative 4 (200 µM) | 99.34 |

From FIG. 11 and Table 9, it can be seen that the artemisinic acid and its derivatives can all reduce the content of melanin, while the dihydroartemisinic acid and its derivatives do not reduce the content of melanin. Through a comparison between the artemisinic acid and the derivatives 1-3, it can be known that the artemisinic acid derivatives 2-3 provided by the present application have a better melanin inhibition effect than the artemisinic acid and an artemisinic acid metal salt, and the above derivatives have lower effective concentrations and higher safety. Further, the derivative 2 provided by the present application has no significant difference in whitening effect with α-arbutin as a positive control at a concentration of 200 µM. Thus, it can be known that the artemisinic acid derivatives provided by the present application further enhance whitening activity on the basis of improving safety.

### Test Example 5

### Evaluation of whitening activity of artemisinic acid and its derivatives (animal experiment)

Test samples: artemisinic acid, dihydroartemisinic acid, and derivatives 1-5.

### Test method:

Brown skin parts of coloured guinea pigs were selected for administration by application. After modeling by UVB irradiation (at a UVB lamp tube irradiation wavelength of 310 nm and a total cumulative irradiation amount of about 2,000 mJ/cm²), dorsal hair of each guinea pig was shaved to divide the back into two depilated areas with a size of 2 cm × 2 cm. One area was used as a drug administration area, onto which 50 µL of 25 mg/mL solutions of the artemisinic acid and its derivatives were applied each time using a pipette for 2 times a day. The other area was used as a blank control area, onto which 50 µL of a 1/15 M phosphate buffer (pH=6.8) was applied each time. The guinea pigs were shaved with a shaver before each administration. After continuous administration for 30 days, skin tissues were selected, sectioned, stained, and subjected to optical density analysis. Results of optical density/melanocyte area values and optical density/section area values of skin sections with L-DOPA staining or ammoniacal silver staining are shown in Table 10.

**Table 10**

| Staining method | Optical density/melanocyte area | | | Optical density/section area | | |
|---|---|---|---|---|---|---|
| | Blank control | Administration | Change rate/% | Blank control | Administration | Change rate/% |
| L-DOPA staining (artemisinic acid) | 0.323 ± 0.034 | 0.203 ± 0.015*** | 37.15 | 0.074 ± 0.002 | 0.043 ± 0.002*** | 41.89 |
| Ammoniacal silver staining (artemisinic acid) | 0.367 ± 0.024 | 0.278 ± 0.011* | 24.25 | 0.123 ± 0.013 | 0.079 ± 0.017** | 35.77 |
| L-DOPA staining (derivative 1) | 0.316 ± 0.022 | 0.198 ± 0.013*** | 37.34 | 0.068 ± 0.005 | 0.032 ± 0.007*** | 52.94 |
| Ammoniacal silver staining (derivative 1) | 0.358 ± 0.014 | 0.245 ± 0.009* | 31.56 | 0.126 ± 0.033 | 0.067 ± 0.019** | 46.82 |
| L-DOPA staining (derivative 2) | 0.302 ± 0.025 | 0.187 ± 0.014*** | 38.07 | 0.072 ± 0.005 | 0.040 ± 0.003*** | 44.44 |
| Ammoniacal silver staining (derivative 2) | 0.328 ± 0.031 | 0.254 ± 0.015* | 22.56 | 0.112 ± 0.013 | 0.073 ± 0.017** | 34.82 |
| L-DOPA staining (derivative 3) | 0.312 ± 0.025 | 0.186 ± 0.012*** | 40.38 | 0.078 ± 0.003 | 0.050 ± 0.007*** | 35.89 |
| Ammoniacal silver staining (derivative 3) | 0.319 ± 0.013 | 0.264 ± 0.017* | 17.24 | 0.118 ± 0.015 | 0.089 ± 0.023** | 24.57 |
| L-DOPA staining (derivative 4) | 0.320 ± 0.021 | 0.314 ± 0.015 | 1.87 | 0.074 ± 0.013 | 0.067 ± 0.005 | 9.45 |
| Ammoniacal silver staining (derivative 4) | 0.309 ± 0.013 | 0.294 ± 0.017 | 4.85 | 0.108 ± 0.015 | 0.112 ± 0.015 | 3.70 |
| L-DOPA staining (derivative 5) | 0.323 ± 0.034 | 0.218 ± 0.015* | 32.51 | 0.074 ± 0.002 | 0.048 ± 0.002* | 35.14 |
| Ammoniacal silver staining (derivative 5) | 0.367 ± 0.024 | 0.262 ± 0.011* | 28.61 | 0.123 ± 0.013 | 0.099 ± 0.017* | 19.51 |

From Table 10, it can be known that after continuous application of the solutions of the artemisinic acid and its derivatives onto the skin of the guinea pigs for 30 days, both the optical density/melanocyte area values and optical density/section area values of the skin sections with the L-DOPA staining or the ammoniacal silver staining are obviously decreased compared with a control group, and statistical data have significant differences, indicating that the artemisinic acid and its derivatives can effectively inhibit the generation of melanin in the skin and have an obvious skin whitening effect. Moreover, the derivatives 2-3 have better whitening activity than the artemisinic acid or an artemisinic acid metal salt, while the dihydroartemisinic acid and its derivatives do not effectively inhibit the generation of melanin.

### Test Example 6

### Experiment of artemisinic acid and its derivatives on growth of mouse melanoma cells

Test samples: artemisinic acid, dihydroartemisinic acid, and derivatives 1-7.

### Test method:

B16F10 cells in a state of an exponential growth phase were selected, digested with trypsin, and inoculated into a 6-well plate at a cell density of 7×10⁴ cells/mL at 2 mL/well. After adherent culture overnight, a culture medium was replaced, the cells were treated with the artemisinic acid, the dihydroartemisinic acid, and their derivatives for 48 h, with 3 replicate wells for each sample. The cultured cells were observed and photographed. The culture medium was discarded, and the cells were washed once with PBS. Cell culture dishes were placed on an ice plate, 330 µL of a non-denaturing cell lysis buffer (containing 1 mM of PMSF) was added into each dish for lysis at 4°C for 20 min, and the cells were collected. Centrifugation was performed at 13,000 rpm for 10 min to obtain supernatants in centrifuge tubes to serve as cellular proteins. Protein contents were measured using a BCA protein content assay kit, 3 replicate wells and a blank well were set, and an OD value was measured at 562 nm.

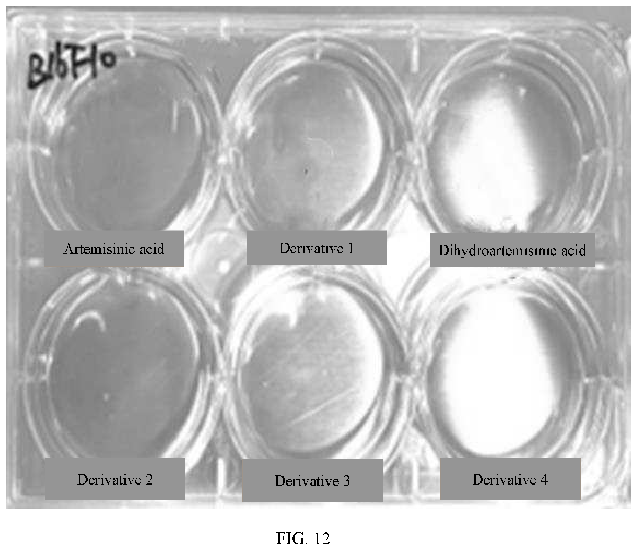

Test results are shown in FIG. 12 and Table 11.

**Table 11**

| Sample name | Protein content/µg/mL |
|---|---|
| Derivative 2 | 722 |
| Derivative 3 | 845 |
| Derivative 5 | 818 |
| Derivative 6 | 872 |
| Derivative 7 | 874 |
| Artemisinic acid | 772 |
| Dihydroartemisinic acid | 800 |
| Derivative 1 | 911 |
| Derivative 4 | 831 |

As shown in FIG. 12, culture media of the artemisinic acid and the derivative 2 are pink in colour, indicating low consumption of nutrients, and indirectly indicating a decrease in cell number. It is indicated that growth of mouse melanoma cells can be inhibited, which is also confirmed by combining protein content measurement data. Therefore, the artemisinic acid and its derivatives have a certain anti-tumor effect.

### Test Example 7

### Evaluation of anti-inflammatory activity of artemisinic acid and its derivatives

Test samples: artemisinic acid and derivatives 1-7.

### Test method:

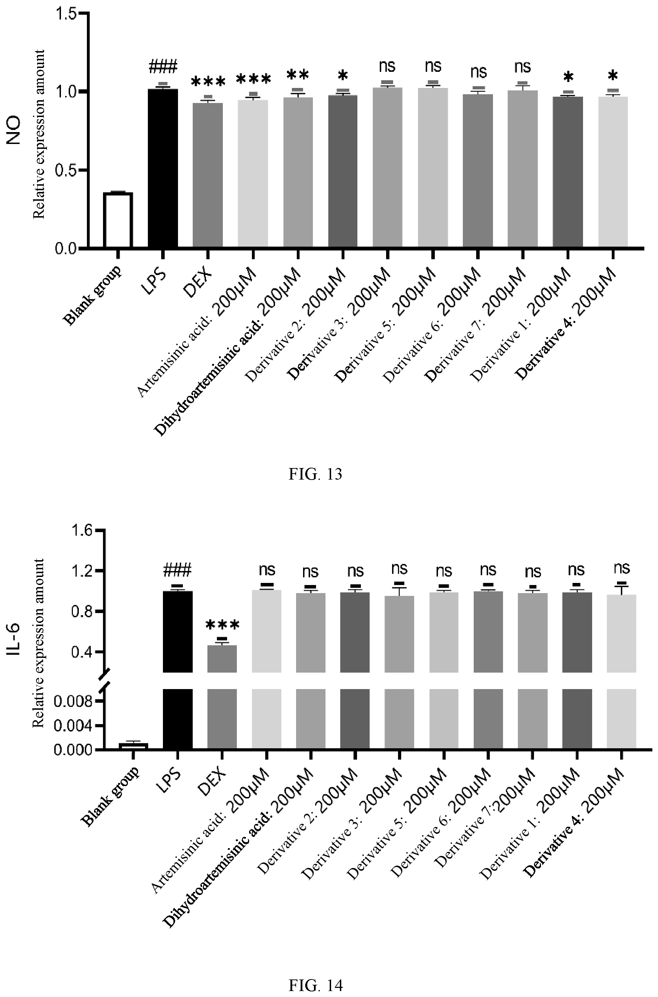

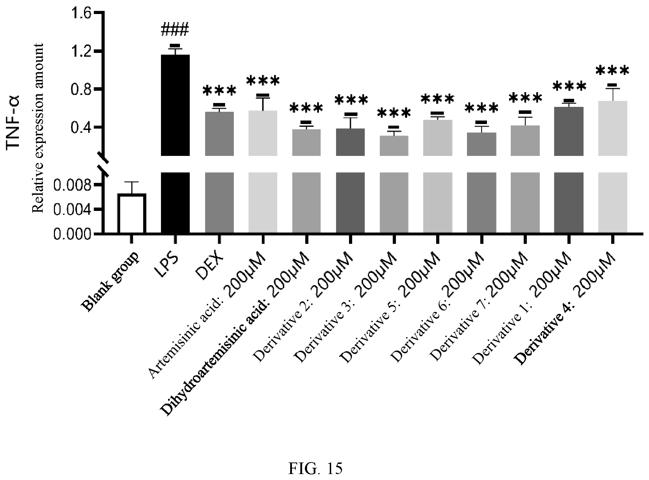

RAW264.7 cells with good morphology in a logarithmic growth phase were selected, inoculated into a 24-well plate, and incubated in an incubator for 24 h. A blank control group, an LPS-induced stimulation group, a positive control group (dexamethasone, DEX), and a sample group were set. The cells were incubated in a 5% CO₂ incubator at 37°C for 2 h. Except for the blank control group, LPS was added into each well, and the cells were incubated in the incubator for 24 h. Cell supernatants were detected according to instruction steps of an ELISA kit and a nitric oxide assay kit, and contents of TNF-α, IL-6, and NO in the collected cell supernatants were detected, respectively. Results are shown in FIG. 13, FIG. 14, FIG. 15, and Table 12.

**Table 12**

| | Relative expression amount of NO | Relative expression amount of IL-6 | Relative expression amount of TNF-α |
|---|---|---|---|
| Blank group | 0.36 | 0.001 | 0.006 |
| LPS | 1.01 | 1.00 | 1.15 |
| DEX | 0.92 | 0.47 | 0.56 |
| Derivative 2 (200 µM) | 0.97 | 0.98 | 0.38 |
| Derivative 3 (200 µM) | 1.02 | 0.95 | 0.31 |
| Derivative 5 (200 µM) | 1.02 | 0.98 | 0.47 |
| Derivative 6 (200 µM) | 0.98 | 0.99 | 0.34 |
| Derivative 7 (200 µM) | 1.01 | 0.98 | 0.42 |
| Artemisinic acid (200 µM) | 0.94 | 1.01 | 0.57 |
| Dihydroartemisinic acid (200 µM) | 0.96 | 0.98 | 0.38 |
| Derivative 1 (200 µM) | 0.97 | 0.98 | 0.61 |
| Derivative 4 (200 µM) | 0.96 | 0.96 | 0.68 |

According to the test results shown in FIG. 13, FIG. 14, FIG. 15, and Table 12, the artemisinic acid and its derivatives have almost no effect on the two targets of NO and IL-6 among anti-inflammatory action targets, but have a significant and good effect on the TNF-α target. Moreover, the derivatives show a better trend. The results also show that the artemisinic acid has a certain effect in alleviating skin inflammation, speculating that it may also assist in whitening through an anti-inflammatory pathway.

Although the dihydroartemisinic acid and its derivatives cannot reduce melanin, they also have a certain anti-inflammatory effect.

### Test Example 8

### Solubility test

Test samples: artemisinic acid, dihydroartemisinic acid, and derivatives 2-3 and 5-7.

The solubility of the artemisinic acid, the dihydroartemisinic acid, and the derivatives 2-3 and 5-7 in water at 25°C were tested by a gravimetric method. Test results are shown in Table 13.

**Table 13**

| Sample | Solubility (g/100 g of water) |
|---|---|
| Derivative 2 | 6.5 |
| Derivative 3 | 10.2 |
| Derivative 5 | 7.2 |
| Derivative 6 | 6.3 |
| Derivative 7 | 9.6 |
| Artemisinic acid | < 0.01 |
| Dihydroartemisinic acid | < 0.01 |

According to the test results, it can be obtained that the artemisinic acid derivatives provided by the present application have higher solubility in water, thus solving the problem of poor water solubility of the artemisinic acid or the dihydroartemisinic acid.

The applicant declares that the above description only shows specific embodiments of the present application, but the scope of protection of the present application is not limited thereto. Those skilled in the art should understand that any alterations or substitutions easily conceived by those skilled in the art within the technical scope disclosed by the present application fall within the scope of protection and the scope of disclosure of the present application.

## Claims

1. An artemisinic acid derivative, **characterized in that** the artemisinic acid derivative comprises: a water-soluble organic salt formed by a reaction of artemisinic acid or dihydroartemisinic acid with a small-molecule basic organic compound, or a water-soluble complex formed by a reaction of artemisinic acid or dihydroartemisinic acid with a small molecule peptide, or a water-soluble salt formed by a reaction of artemisinic acid or dihydroartemisinic acid with a basic metal compound;
wherein the artemisinic acid derivative has the following structures: 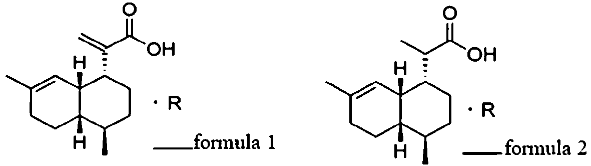 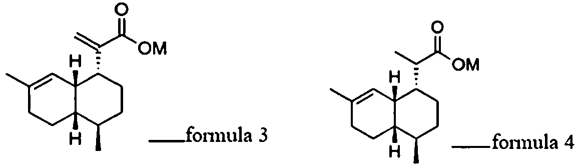
wherein R is selected from a small-molecule basic organic compound or a small molecule peptide, and M is a metal.

2. The artemisinic acid derivative according to claim 1, **characterized in that** the small-molecule basic organic compound comprises a basic amino acid or an alkaloid;
the basic amino acid comprises lysine, arginine, or histidine;
the alkaloid comprises nicotinamide or ligustrazine;
the small molecule peptide comprises glutathione; and
the basic metal compound comprises a sodium salt, a potassium salt, potassium hydroxide, or sodium hydroxide.

3. The artemisinic acid derivative according to claim 1, **characterized in that** the artemisinic acid derivative is selected from any one of the following compounds:

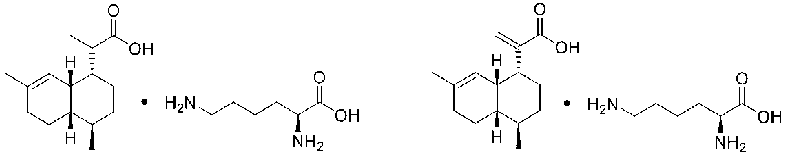

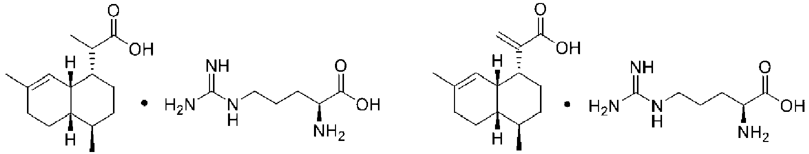

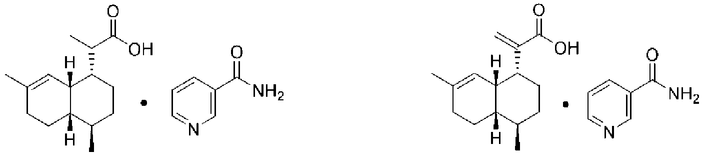

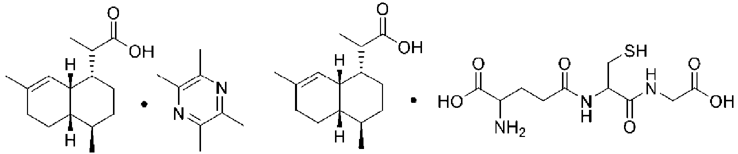

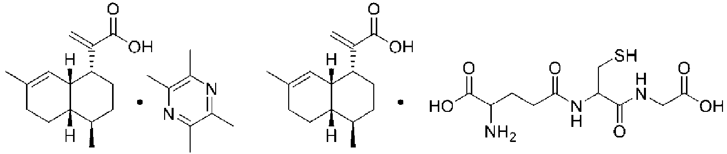

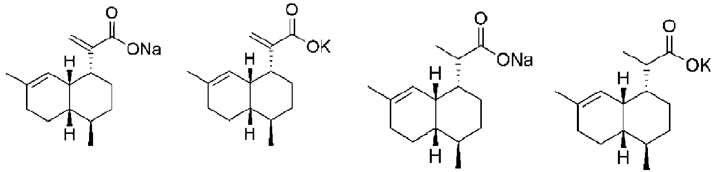

4. A method for preparing the artemisinic acid derivative according to claim 1, **characterized in that** the preparation method comprises: allowing the artemisinic acid or the dihydroartemisinic acid to undergo a reaction with a modifier in water to obtain the artemisinic acid derivative, wherein the modifier is a small-molecule basic organic compound, a small molecule peptide, or a basic metal compound.

5. The method according to claim 4, **characterized in that** a molar ratio of the artemisinic acid or the dihydroartemisinic acid to the modifier is 1:(0.9-1.1); and
a use amount of the water is 5-20 times of a total mass of the artemisinic acid or the dihydroartemisinic acid and the modifier.

6. The method according to claim 4, **characterized in that** a temperature of the reaction is 20-30°C;
a time of the reaction is 30-60 s;
the method further comprises a step of post-treatment after the reaction is completed, and the post-treatment comprises filtration and drying; and
the drying comprises any one or a combination of at least two of freeze-drying, spray drying, or vacuum drying.

7. An application of the artemisinic acid derivative according to claim 1 in a skin care product or a pharmaceutical product, **characterized in that**
the skin care product comprises an essence liquid, a facial mask, a microneedle, an emulsion, a lyophilized powder, or a face cream;
a mass percentage of the artemisinic acid derivative in the skin care product is 1-40%; and
a dosage form of the pharmaceutical product comprises a tablet, a capsule, a granule, an injection, a spray, or a film.

8. An emulsion, **characterized in that** the emulsion comprises one or a combination of at least two of the artemisinic acid derivative according to claim 1, an emollient, an emulsifier, a thickener, a stabilizer, a preservative, and an antioxidant.

9. A lyophilized powder, **characterized in that** the lyophilized powder comprises one or a combination of at least two of the artemisinic acid derivative according to claim 1, and a humectant.

10. A tyrosinase inhibitor, **characterized in that** the tyrosinase inhibitor comprises any one or a combination of at least two of the artemisinic acid derivative according to claim 1.
